# EUROPÄISCHE PATENTSCHRIFT

(11) **EP 1 681 022 B1**
(45) Veröffentlichungstag und Bekanntmachung des Hinweises auf die Patenterteilung: **31.10.2007**
(21) Anmeldenummer: 06004890.7
(22) Anmeldetag: 08.09.2004
(51) Int. Cl.: A61B 17/32, A61B 17/22, A61B 17/34

(54) **Medizinisches Instrument zum Schneiden von biologischem und insbesondere menschlichem Gewebe**
Medical instrument for cutting biological tissue
Instrument médical pour couper des tissues biologiques

(30) Priorität: 11.12.2003 DE 10358279
(43) Veröffentlichungstag der Anmeldung: 19.07.2006
(62) Teilanmeldung aus: 04764940.5
(73) Patentinhaber: Karl Storz GmbH & Co. KG, 78532 Tuttlingen (DE)
(72) Erfinder: Seeh, Daniel, 78194 Immendingen (DE)
(74) Vertreter: Hofmeister, Frank

(56) Entgegenhaltungen:
- EP-A- 0 769 278
- DE-C1- 4 306 205
- US-B1- 6 439 541

## Beschreibung

Die Erfindung betrifft einmedizinisches Instrument zum Schneiden von biologischem und insbesondere menschlichem Gewebe mit einem über einen Motor um seine Längsachse drehbaren hohlen Schneidrohr, an dessen distalem Ende mindestens eine Schneide angeordnet ist, sowie zur Aufnahme mindestens eines weiteren medizinischen Instruments, das in das hohle Schneidrohr einsetzbar ist, wobei das Schneidrohr in einem sich entlang der Längsachse erstreckenden zentralen Kanal gelagert ist und mindestens ein Dichtungsmittel vorgesehen ist, um den zentralen Kanal gasdicht abzudichten.

Derartige, Morcellatoren genannte medizinische Instrumente werden bei endoskopischen Eingriffen verwendet, die die Entfernung großer Gewebeanteile notwendig machen. Diese Morcellatoren bestehen aus einem angetriebenen Schneidrohr, das direkt in den Körper bzw. eine natürliche oder künstliche Körperhöhle eingeführt werden kann. Zur Gewebeentfernung wird durch das Schneidrohr eine Fasszange in den Körper bzw. die Körperhöhle eingeführt, mit der das zu extrahierende Gewebe gefasst wird. Wird die Zange nun zurückgezogen und das Gewebe gegen die Schneidkante des rotierenden Morcellators gepresst, so kann durch dosierten Zug und gegebenenfalls Variation der Drehgeschwindigkeit und Drehrichtung ein zylinderförmiger Gewebeblock ausgeschnitten und durch das Rohr entfernt werden. Selbst große Gewebemengen können so innerhalb weniger Minuten extrahiert werden.

Ein gattungsgemäßes medizinisches Instrument ist beispielsweise aus der 5 618 296 A bekannt. Bei dieser bekannten Vorrichtung ist der Motor als getriebelos auf dem Schneidrohr angeordneter Hohlwellenmotor ausgebildet. Nachteilig bei dieser bekannten Konstruktion ist, dass durch das Aufsetzen des Motors auf das Schneidrohr zwar die Verluste bei der Leistungsübertragung reduziert werden konnten, dafür aber die Demontage zu Reinigungs- und Wartungszwecken deutlich erschwert wurde.

US-B1-6 638 238 offenbart ein medizinisches Instrument gemäß dem Oberbegriff des Anspruchs 1.

Weitere medizinische Instrumente zum Schneiden von biologischem und insbesondere menschlichem Gewebe sind beispielsweise aus der EP-B1-0 555 803 und der EP-A1-0 806 183 bekannt. Bei diesen bekannten Morcellatoren erfolgt der rotierende Antrieb des Schneidrohres über ein zwischen Motor und Schneidrohr zwischengeschaltetes Zahnrad- und/oder Schneckengetriebe. Die Verwendung dieser der Drehmomentübertragung dienenden Getriebe erzeugen im Betrieb jedoch hohe Leistungsverluste der eigentlichen Motorleistung. Darüber hinaus sind die Getriebe teilweise sehr kompliziert aufgebaut und somit auch sehr teuer.

Aus der EP-A2-0 769 278 ist darüber hinaus ein modulares Trokarsystem bekannt, welches zum Abdichten des hohlen zentralen Kanals des Morcellatorschaftes ein in dem hohlen Kanal angeordnetes Klappenventil aufweist. Nachteilig bei dieser bekannten Konstruktion ist, dass die Klappenflügel des Klappenventils beim Einführen des Schneidrohres in den hohlen zentralen Kanal durch die Schneidkante des Schneidrohres beschädigt werden können.

Davon ausgehend liegt der Erfindung die **Aufgabe** zugrunde, ein medizinisches Instrument der eingangs genannten Art so zu verbessern, der zentrale Kanal auch bei eingeführtem Schneidrohr zuverlässig abdichtbar ist.

Die **Lösung** dieser Aufgabenstellung ist erfindungsgemäß dadurch gekennzeichnet, dass das mindestens eine Dichtungsmittel als in den zentralen Kanal einsetzbares Klappenventil ausgebildet ist, dessen zwei zum distalen Ende des zentralen Kanals weisenden Klappenflügel unter einem Winkel aneinander anliegen, der flacher ist als der Winkel der Schneide am distalen Ende des in den zentralen Kanal einsetzbaren Schneidrohres.

Die Erfindung betrifft weiterhin ein medizinisches Instrument zum Schneiden von biologischem und insbesondere menschlichem Gewebe mit einem sich entlang der Längsachse erstreckenden zentralen Kanal zur Aufnahme eines über einen Motor um seine Längsachse drehbaren hohlen Schneidrohres, an dessen distalem Ende mindestens eine Schneide angeordnet ist, sowie zur Aufnahme mindestens eines weiteren medizinischen Instruments, das in das hohle Schneidrohr einsetzbar ist, wobei mindestens ein Dichtungsmittel vorgesehen ist, um den zentralen Kanal gasdicht abzudichten.

Da es bei endoskopischen Operationen des Bauchraums üblich ist, den Bauchraum zur Erweiterung des Operationsraums und Ausbildung eines Pneumoperitoneums mit Gas zu befüllen, ist der hohle zentrale Kanal über mindestens ein Dichtungsmittel verschließbar, damit das Gas nicht beispielsweise über eine Trokarhülse aus dem Bauchraum entweichen kann.

Gemäß einer ersten Ausführungsform der Erfindung wird vorgeschlagen, dass das mindestens eine Dichtungsmittel als in den zentralen Kanal einsetzbares Klappenventil ausgebildet ist, dessen zwei zum distalen Ende des zentralen Kanals weisenden Klappenflügel unter einem Winkel aneinander anliegen, der flacher ist als der Winkel der Schneide am distalen Ende des in den zentralen Kanal einsetzbaren Schneidrohres.

Aufgrund der Wahl dieses Winkels der schnabelförmig aneinander anliegenden Klappenflügel wird gewährleistet, dass beim Durchschieben des Schneidrohres nicht die Schneide des Schneidrohres gegen die Klappenflügel stößt, um die Klappenflügel zu öffnen, sondern die proximalen Enden der Schneidenabschrägung die Klappenflügel vorrangig in radialer Richtung nach außen öffnen.

Weiterhin wird mit der Erfindung vorgeschlagen, dass als zusätzliches Dichtungsmittel am proximalen Ende des zentralen Kanals eine Dichtungskappe festlegbar ist. diese Art der Ausbildung des Dichtungsmittels stellt eine besonders einfache und leicht zu montierende Ausführungsform dar, wobei die Dichtungskappe vorteilhafterweise als aus einem autoklavierbaren Material bestehendes Einmal-Ventil ausgebildet ist.

Die Dichtungskappe ist gemäß einer praktischen Ausführungsform der Erfindung als von dem in den zentralen Kanal einsetzbaren medizinischen Instrument durchstoßbare Membran ausgebildet ist. Bei dieser als Einmalventil ausgebildeten Dichtungskappe legt sich das durchstoßene Membranmaterial abdichtend an die Außenseite des in den zentralen Kanals eingesetzten medizinischen Instruments an.

Gemäß einer alternativen Ausführungsform der Erfindung wird vorgeschlagen, dass in der Dichtungskappe eine durch das in den zentralen Kanal einsetzbaren medizinischen Instrument aufweitbare Öffnung ausgebildet ist.

Schließlich wird mit der Erfindung vorgeschlagen, dass das Instrument zur Ausbildung des zentralen Kanals zumindest eine distalseitig angeordnete Trokarhülse, einen mit einer zentralen Aufnahme für das anzutreibende Schneidrohr versehenen, als Hohlwellenmotor ausgebildeten Motor sowie eine proximalseitig angeordnete, mit einer zentralen Durchgangsbohrung versehene Handhabe umfasst.

Das Führen des erfindungsgemäßen Instruments kann für den Operateur dadurch erleichtert werden, dass an der Handhabe ein zusätzlicher, abgewinkelter Handgriff festlegbar ist. Vorzugsweise ist dieser zusätzliche Handgriff um 90° gegenüber der Instrumentenlängsachse abgewinkelt und gewichtsoptimiert ausgebildet.

Weitere Merkmale und Vorteile der Erfindung ergeben sich anhand der zugehörigen Zeichnung, in der verschiedene Ausführungsbeispiele eines erfindungsgemäßen medizinischen Instruments zum Schneiden von biologischem und insbesondere menschlichem Gewebe nur beispielhaft dargestellt sind. In der Zeichnung zeigt:
- Fig. 1: einen ausschnittweisen schematischen Längsschnitt durch eine erste Ausführungsform eines erfindungsgemäßen medizinischen Instruments;
- Fig. 2: eine vergrößerte Detailansicht einer erfindungsgemäßen Kopplung zwischen Schneidrohr und Motor gemäß einer zweiten Ausführungsform der Erfindung;
- Fig. 3: eine perspektivische Explosionszeichnung einer ersten praktischen Ausführungsform eines erfindungsgemäßen Instruments;
- Fig. 4: eine Seitenansicht des Instruments gemäß Fig. 3 im zusammengebauten Zustand;
- Fig. 5: einen Längsschnitt durch das Instrument gemäß Fig. 4;
- Fig. 6: eine Seitenansicht gemäß Fig. 4, jedoch das Instrument mit eingesetztem zusätzlichem Instrument darstellend;
- Fig. 7: eine perspektivische Explosionszeichnung einer zweiten praktischen Ausführungsform eines erfindungsgemäßen Instruments;
- Fig. 8: einen schematischen Längsschnitt durch ein erfindungsgemäßes Klappenventil in der geschlossenen Stellung;
- Fig. 9: einen Fig. 8 entsprechenden Längsschnitt, jedoch das Klappenventil in der geöffneten Stellung mit eingesetztem Schneidrohr darstellend und
- Fig. 10: eine schematische Draufsicht auf eine erfindungsgemäße Dichtungskappe.

Das in der Abbildung dargestellte, als Morcellator ausgebildete medizinische Instrument zum Schneiden von biologischem und insbesondere menschlichem Gewebe besteht im wesentlichen aus einem in einer Handhabe 1 drehbar gelagerten hohlen Schneidrohr 2 sowie einem Motor 3, über den das Schneidrohr 2 um seine Längsachse 4 drehbar antreibbar ist. Zum Schneiden des Gewebes weist das Schneidrohr 2 am distalseitigen Ende eine Schneide 2a auf. Die Handhabe 1 wird bei der dargestellten Ausführungsform durch ein Motorgehäuse 3a des Motors 3 gebildet.

Wie weiterhin aus der Abbildung ersichtlich, ist das Schneidrohr 2 bei der dargestellten Ausführungsform zumindest in seinem Bereich distalseitig der Handhabe 1 koaxial von einem beispielsweise als Trokarhülse 5 ausgebildeten Außenrohr umgeben, deren distales Ende unter Ausbildung einer Spitze 5a in Längsrichtung schräg verlaufend ausgebildet ist, wobei die Trokarhülse 5 das Schneidrohr 2 zumindest im Bereich der Spitze 5a distalseitig überragt. Neben dem Offenhalten des Zugangs zum Operationsgebiet dient die Trokarhülse 5 dazu, die Schneide 2a des Schneidrohres 2 beispielsweise beim Einführen des Morcellators in das Operationsgebiet zumindest teilweise abdecken zu können, um keine unbeabsichtigten Verletzungen an zu schonendem Gewebe zu verursachen. Vorteilhafterweise ist die Trokarhülse 5 so ausgebildet, dass sie zwischen einer die Schneide 2a verdeckenden und eine die Schneide 2a freigebenden Stellung in Richtung der Längsachse 4 des Schneidrohres 2 verlagerbar ist.

Selbstverständlich ist es auch möglich, das Schneidrohr 2 so auszubilden, dass es zwischen einer die Spitze 5a der Trokarhülse 5 überragenden und einer hinter die Spitze 5a der Trokarhülse 5 zurückgezogenen Stellung in Richtung der Längsachse 4 des Instruments verlagerbar ist.

Neben der dargestellten Verwendung einer mit einer Spitze 5a versehenen schrägen oder spitzen Trokarhülse 5 ist es selbstverständlich auch möglich, einen wie voranstehend beschriebenen Morcellator mit einer geraden oder stumpfen Trokarhülse 5 zu versehen, die distalseitig einen geraden Abschluss aufweist.

Um das Schneidrohr 2 möglichst reibungsarm drehbar in der Handhabe 1 (dem Motorgehäuse 3a) zu lagern, sind bei dieser Ausführungsform zwei als Kugellager ausgebildete Lager 6 vorgesehen, von denen zumindest eines vorzugsweise als Schrägkugellager oder Kegelrollenlager ausgebildet ist, um auch in axialer Richtung wirkende Kräfte aufnehmen zu können.

Der Motor 3 zum rotierenden Antrieb des Schneidrohres 2 ist als im Bereich des als Handhabe 1 ausgebildeten Motorgehäuses 3a auf dem Schneidrohr 2 angeordneter Hohlwellenmotor ausgebildet, über den das Schneidrohr 2 direkt, das heißt getriebelos antreibbar ist.

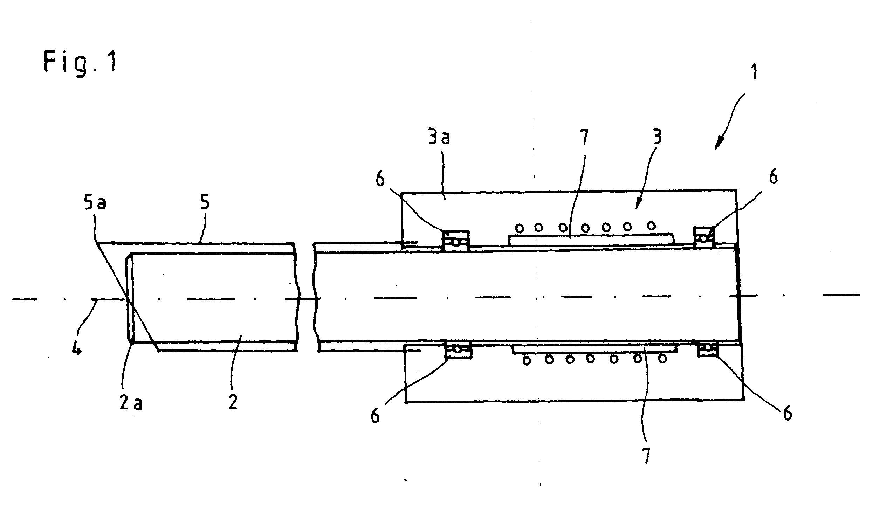

Beim in Fig. 1 dargestellten Ausführungsbeispiel ist der Hohlwellenmotor als Außenläufer-Motor ausgebildet, wobei ein mit der Wicklung versehener Rotor 7 das als Stator des Motors 3 dienende aus einem magnetisierbaren Material hergestellte Schneidrohr 2 koaxial umgibt.

Beim Betrieb eines solchermaßen ausgebildeten Morcellators bildet der mit einer Hand die Handhabe 1 (das Motorgehäuse 3a) festhaltende Bediener des Morcellators die Drehmomentstütze für den Motor 3. Da der Rotor 7 in der Handhabe 1 angeordnet ist, bewirkt dieses Festhalten des Rotors 7 des Außenläufer-Motors, dass nunmehr das Schneidrohr 2 den rotierenden Teil des Motors 3 bildet und sich um seine Längsachse 4 dreht.

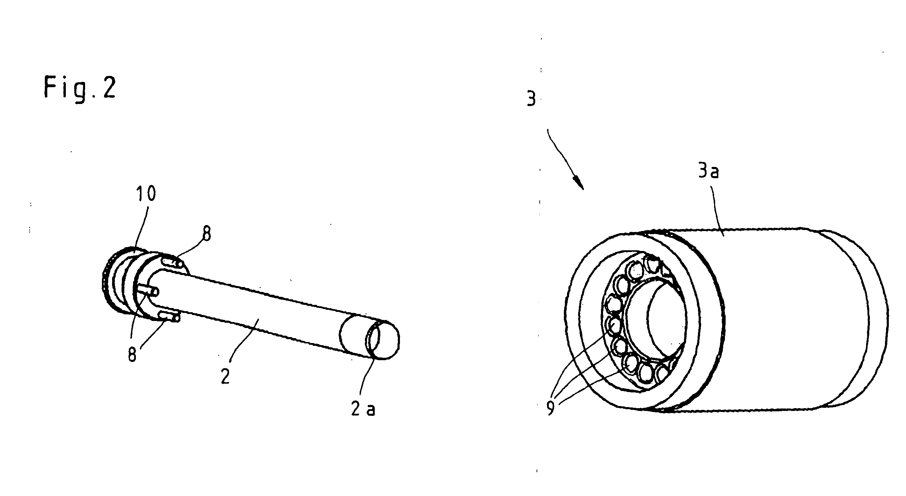

Fig. 2 zeigt eine alternative Ausführungsform des Schneidrohrantriebs. Bei dieser Ausgestaltungsform ist der Motor 3 wiederum als auf dem Schneidrohr 2 angeordneter Hohlwellenmotor ausgebildet, jedoch sind der Motor 3 und das Schneidrohr 2 über in Axialrichtung des Schneidrohres 2 verlaufende, am Schneidrohr 2 angeordnete Stifte 8 miteinander form- und kraftschlüssig miteinander gekoppelt, die in entsprechende Ausnehmungen 9 am Motor 3 einsteckbar sind. Wie weiterhin aus Fig. 2 ersichtlich, sind die Stifte 8 an einem auf dem Schneidrohr 2 angeordneten Adapter 10 angeordnet.

Um eine gleichmäßige und im wesentliche unwuchtfreie Kopplung zu ermöglichen, sind am Schneidrohr 2 bzw. am Adapter 10 vier gleichmäßig um die Längsachse des Schneidrohres 2 verteilt angeordnete Stifte 8 angeordnet. Die Montage der Kopplung der beiden Bauteile 2 und 3 wird dadurch vereinfacht werden, dass am Motor 3 mehr als vier Ausnehmungen 9 zur Aufnahme der Stifte 8 ausgebildet sind, damit die Bauteile 2 und 3 in verschiedenen Positionen zueinander miteinander koppelbar sind.

Selbstverständlich ist es auch möglich, die Kopplung zwischen dem Motor 3 und dem Schneidrohr 2 so auszugestalten, dass die Stifte 8 am Motor 3 und die Ausnehmungen 9 zur Aufnahme der Stifte 8 am Schneidrohr 2 oder dem auf dem Schneidrohr 2 festgelegten Adapter 10 angeordnet sind. Ebenso sind auch Ausführungen mit weniger, insbesondere drei Stiften 8, oder mit mehr als vier Stiften 8 möglich.

Die Verwendung des direkt auf dem Schneidrohr 2 angeordneten Hohlwellenmotors stellt eine besonders platzsparende und einfach zu herzustellende und zu montierende Form des Schneidrohrantriebs dar. Darüber hinaus können durch den getriebelosen direkten Antrieb des Schneidrohres 2 die Leistungsverluste bei der Übertragung des Motordrehmoments auf das Schneidrohr 2 weitestgehend minimiert werden.

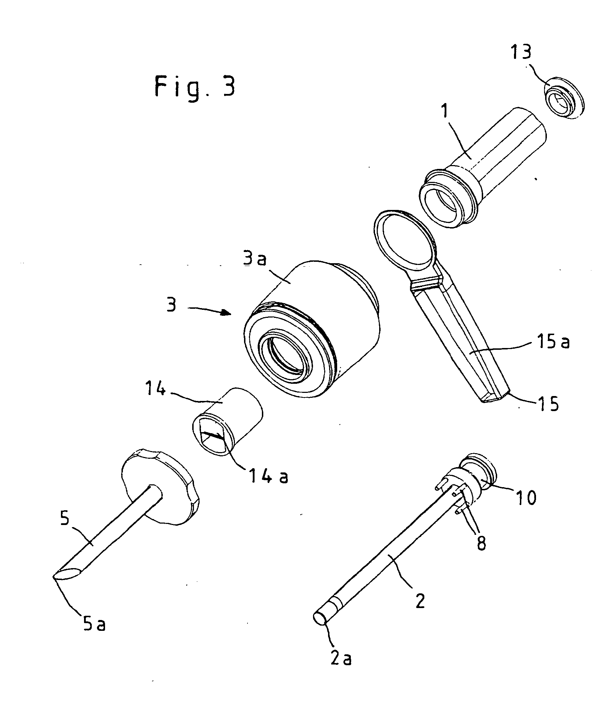

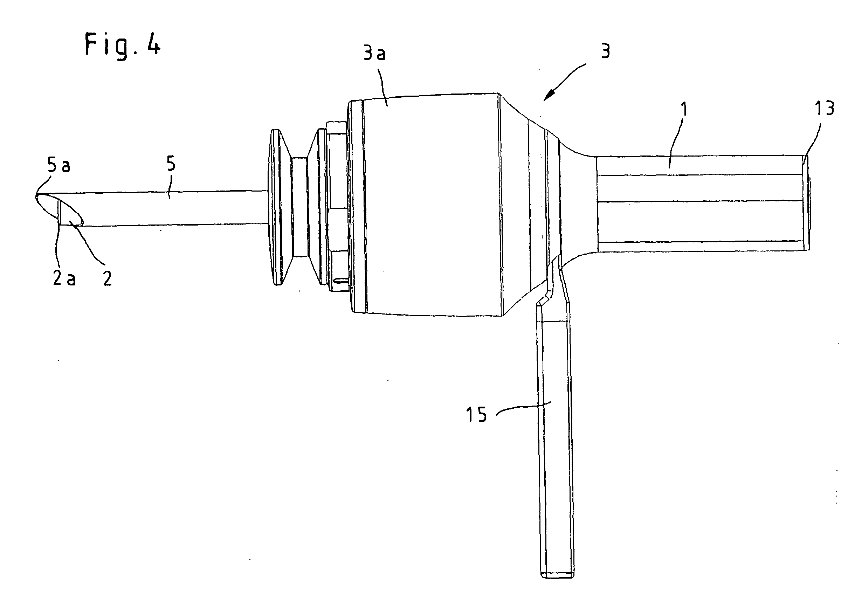

In den Abbildungen Fig. 3 bis Fig. 6 ist eine erste praktische Ausführungsform zur Ausbildung eines als Morcellator für laparoskopische Eingriffe ausgebildeten medizinischen Instruments zum Schneiden von biologischem und insbesondere menschlichem Gewebe. Wie insbesondere aus der Explosionsdarstellung gemäß Fig. 3 ersichtlich, besteht der dargestellte Morcellator im wesentlichen aus einem gleichzeitig das Morcellatorgehäuse bildenden Motorgehäuse 3a des als Hohlwellenmotor ausgebildeten Motors 3, der proximalseitig am Motorgehäuse 3a angeordneten Handhabe 1, der distalseitigen Trokarhülse 5 sowie dem im Inneren des Motorgehäuses 3a sowie der Trokarhülse 5 gelagerten Schneidrohr 2.

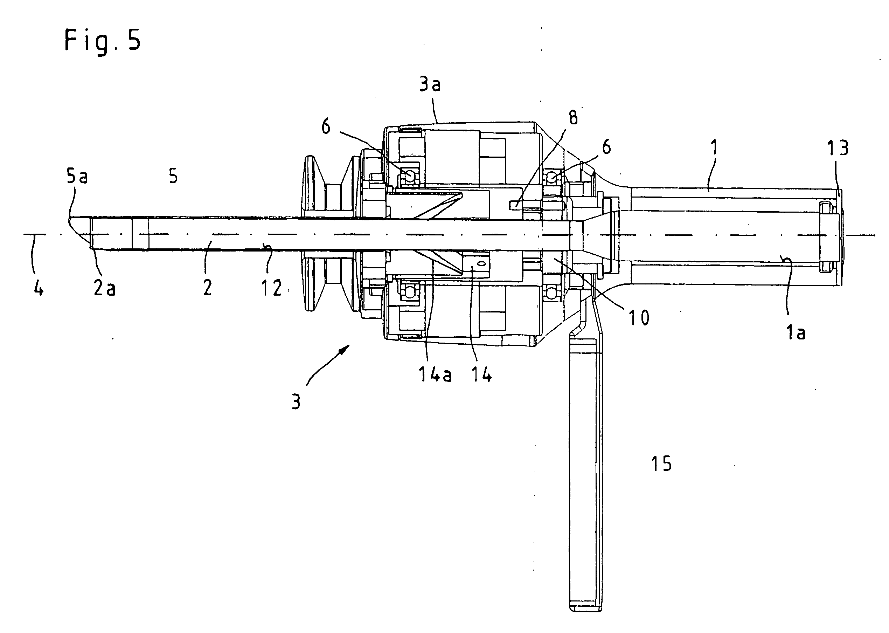

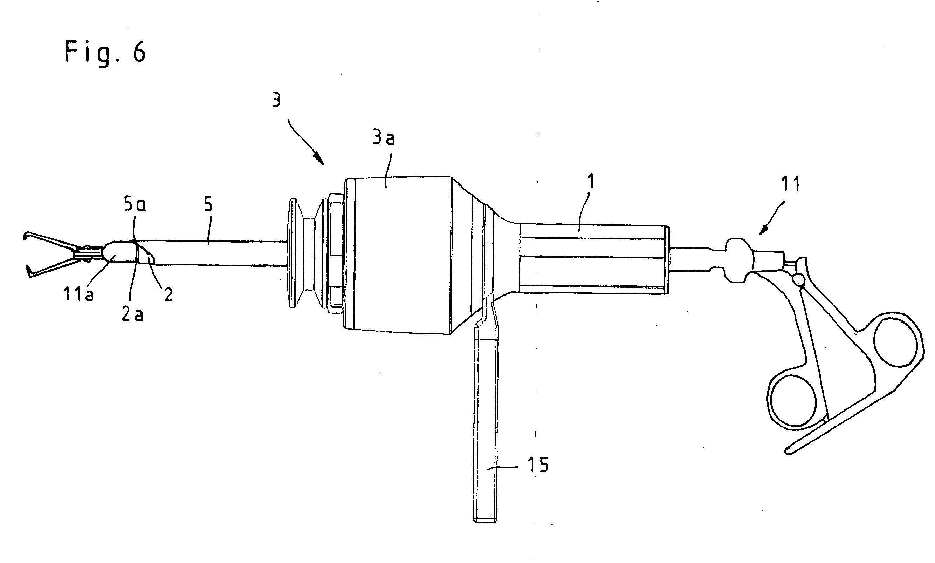

Zur Aufnahme eines weiteren medizinischen Instruments 11, wie dies in Fig. 6 dargestellt ist, weist der Morcellator einen sich entlang der Längsachse 4 des Morcellators erstreckenden zentralen Kanal 12 auf, zu dessen Ausbildung die Handhabe 1 eine zentrale Durchgangsbohrung 1a aufweist. Dieser zentrale Kanal 12 ist insbesondere der Schnittdarstellung gemäß Fig. 5 zu entnehmen.

Da es bei endoskopischen Operationen des Bauchraums üblich ist, den Bauchraum zur Erweiterung des Operationsraums und Ausbildung eines Pneumoperitoneums mit Gas zu befüllen, ist der hohle zentrale Kanal 12 über mindestens ein Dichtungsmittel verschließbar, damit das Gas nicht beispielsweise über die Trokarhülse 5 und den zentralen Kanal 12 aus dem Bauchraum entweichen kann.

Bei der in den Abbildungen Fig. 3 bis Fig. 6 dargestellten Ausführungsform sind zur gasdichten Abdichtung des zentralen Kanals 12 zwei Dichtungsmittel vorgesehen, nämlich einerseits eine am proximalen Ende des zentralen Kanals 12 festlegbare Dichtungskappe 13 sowie ein in den zentralen Kanal 12 einsetzbares Klappenventil 14. Beide Dichtungsmittel 13 und 14 sind unabhängig voneinander und in Kombination miteinander verwendbar.

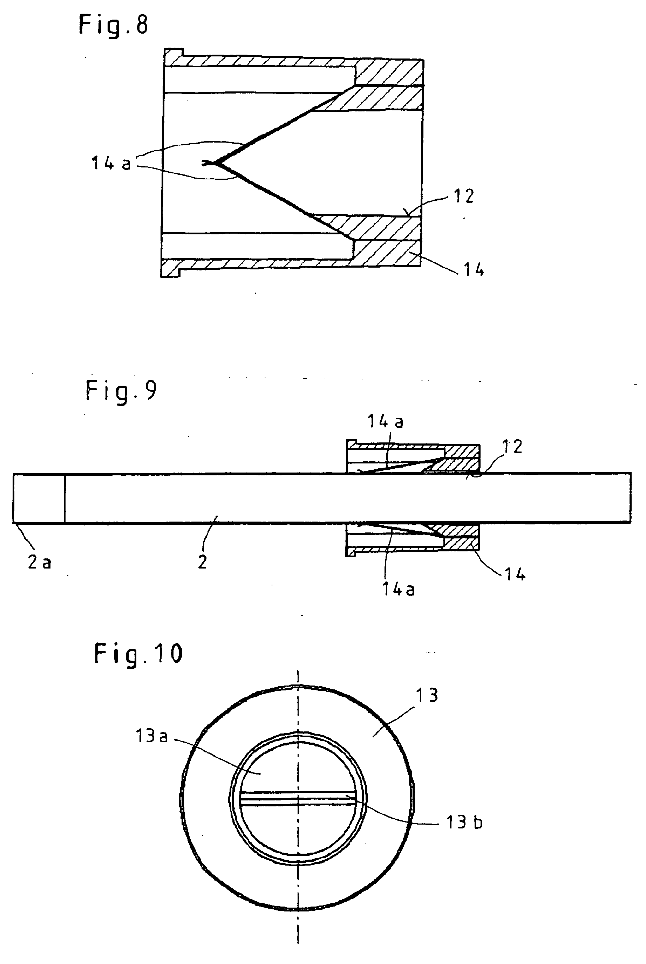

Der Aufbau und die Arbeitsweise des Klappenventils 14 sind insbesondere den schematischen Schnittdarstellungen gemäß Fig. 8 und 9 sowie dem Längsschnitt gemäß Fig. 5 zu entnehmen. Wie aus der Darstellung gemäß Fig. 8 ersichtlich, weist das Klappenventil 14 zwei zum distalen Ende des zentralen Kanals 12 weisende, Klappenflügel 14a auf, die in der geschlossenen Stellung (Fig. 8), also bei nicht in den zentralen Kanal 12 eingesetztem bzw. daraus herausgezogenem Schneidrohr 2, den zentralen Kanal 12 abdichtend fest aneinander anliegen.

Um zu verhindern, dass beim Durchschieben des Schneidrohres 2 durch den zentralen Kanal 12 des Klappenventils 14, wie dies in Fig. 9 dargestellt ist, die Schneide 2a des Schneidrohres 2 mit der scharfen vorderen Schneidkante gegen die Klappenflügel 14a stößt und diese beschädigt, ist der Winkel, unter dem die beiden Klappenflügel 14a des Klappenventils 14 aneinander anliegen, flacher ausgebildet als der Winkel der Schneide 2a am distalen Ende des Schneidrohres 2. Aufgrund der Wahl dieses Winkels der schnabelförmig aneinander anliegenden Klappenflügel 14a wird gewährleistet, dass beim Einführen des Schneidrohres 2 in das Klappenventil 14 die proximalen Enden der Schneidenabschrägung der Schneide 2a des Schneidrohres 2 die Klappenflügel 14a vorrangig in radialer Richtung nach außen öffnen. Schnabelförmig Ausbildung der Klappenflügel 14a bedeutet hierbei, dass die distalen, aneinander anliegenden Enden der Klappenflügel 14a von der Instrumentenachse 4 weg nach außen gekrümmt sind und im Bereich dieser Krümmungen aneinander anliegen.

Bei der in Fig. 9 dargestellten Offenstellung des Klappenventils 14 dichtet das in den zentralen Kanal 12 eingesetzte Schneidrohr 2 den zentralen Kanal 12 ab, da, zumindest im Bereich des Klappenventils 14, der Außendurchmesser des Schneidrohres 2 im wesentlichen dem Innendurchmesser des zentralen Kanals 12 entspricht.

Eine weitere Möglichkeit den zentralen Kanal 12 im Bereich des Schneidrohres 2 gasdicht abzudichten besteht darin, im Bereich des distalen Endes des in das Schneidrohr 2 eingesetzten weiteren medizinischen Instruments 11 einen verdickten Bereich 11a an dem eingesetzten Instrument 11 auszubilden, der dem Innendurchmesser des Schneidrohres 2, insbesondere im Bereich der Schneide 2a entspricht, wie dies der Abbildung Fig. 6 zu entnehmen ist.

Neben der Dichtwirkung des verdickten Bereichs 11a ist dieser verdickte Bereich 11a des zusätzlichen medizinischen Instruments 11 als Schneidkantenschutz verwendbar, der beim Einführen des Schneidrohres 2 in ein Operationsgebiet radial bündig mit der Schneidkante der Schneide 2a in Anlage bringbar ist und in axialer Richtung zumindest bündig mit der Schneidkante abschließt oder distalseitig über die Schneidkante 2a hervorsteht, um das Verletzen von zu schonendem Gewebe auszuschließen.

Die Dichtungskappe 13 zum Verschließen des proximalen Endes des zentralen Kanals 12 ist vorteilhafterweise als aus einem autoklavierbaren Material bestehendes Einmal-Ventil ausgebildet, dessen Dichtungsmaterial 13a beim Einschieben des weiteren medizinischen Instruments 11 in den zentralen Kanal 12 abdichtend am Schaft des eingesetzten Instruments 11 anliegt. Bei der in Fig. 10 dargestellten Ausführungsform der Dichtungskappe 13 ist in dem Dichtungsmaterial 13a ein Schlitz 13b ausgebildet, der durch das Einschieben des Instruments 11 aufgeweitet wird.

Gemäß einer nicht dargestellten alternativen Ausführungsform der Dichtungskappe 13 ist das Dichtungsmaterial 13a als von dem in den zentralen Kanal 12 einsetzbaren Instrument 11 durchstoßbare Membran ausgebildet.

Wie weiterhin aus den Abbildungen Fig. 3, 4 und 6 ersichtlich, ist an der Handhabe 1 ein zusätzlicher, abgewinkelter Handgriff 15 festgelegt, um das Führen des Morcellators für den Operateur zu erleichtern. Vorzugsweise ist dieser zusätzliche Handgriff 15, wie dargestellt, um 90° gegenüber der Instrumentenlängsachse 4 abgewinkelt. Selbstverständlich sind je nach Anwendungsgebiet und/oder Wunsch des Operateurs und/oder ergonomischen Gesichtspunkten auch andere Winkelstellungen des zusätzlichen Handgriffs 15 möglich.

Insbesondere bei der Herstellung des zusätzlichen Handgriffs 15 aus Metall ist es zur Verringerung des Gesamtgewichts des Instruments wichtig, den Handgriff 15 gewichtsoptimiert auszubilden. Hierzu ist es beispielsweise möglich, den Handgriff 15 so auszugestalten, dass dieser im mittleren Bereich 15a einen vollständigen Durchbruch oder aber, wie in Fig. 3 dargestellt, einen Rücksprung aufweist.

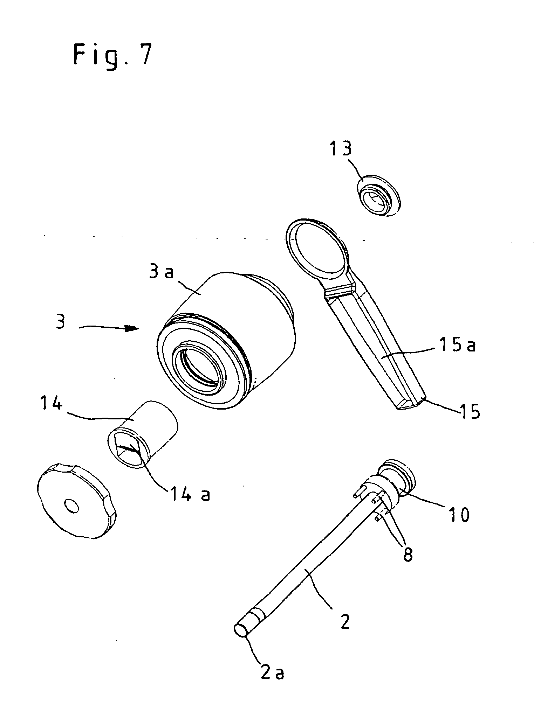

In Fig. 7 ist eine zweite praktische Ausführungsform zur Ausbildung eines als Morcellator ausgebildeten medizinischen Instruments zum Schneiden von biologischem und insbesondere menschlichem Gewebe dargestellt. Dieser für suprazervikale Eingriffe dienende Morcellator unterscheidet sich von dem zuvor insbesondere anhand der Abbildungen Fig. 3, 4 und 6 beschriebenen Morcellator für laparoskopische Eingriffe dadurch, dass dieser keine das Schneidrohr 2 distalseitig umgebende Trokarhülse 5 sowie keine besondere Handhabe 1 aufweist. Statt dessen dient, wie bereits zur Abbildung Fig. 1 beschrieben, das Motorgehäuse 3a als Handhabe 1.

### Bezugszeichenliste

- 1: Handhabe
- 1a: Durchgangsbohrung
- 2: Schneidrohr
- 2a: Schneide
- 3: Motor
- 3a: Motorgehäuse
- 4: Längsachse
- 5: Trokarhülse
- 5a: Spitze
- 6: Lager
- 7: Rotor
- 8: Stift
- 9: Ausnehmung
- 10: Adapter
- 11: medizinisches Instrument
- 11 a: verdickter Bereich
- 12: zentraler Kanal
- 13: Dichtungskappe
- 13a: Dichtungsmaterial
- 13b: Schlitz
- 14: Klappenventil
- 14a: Klappenflügel
- 15: Handgriff
- 15a: mittlerer Bereich

## Patentansprüche

1. Medizinisches Instrument zum Schneiden von biologischem und insbesondere menschlichem Gewebe mit einem über einen Hohlwellenmotor (3) um seine Längsachse (4) drehbaren hohlen Schneidrohr (2), an dessen distalem Ende mindestens eine Schneide (2a) angeordnet ist, sowie zur Aufnahme mindestens eines weiteren medizinischen Instruments (8), das in das hohle Schneidrohr (2) einsetzbar ist, wobei das Schneidrohr (2) in einem sich entlang der Längsachse (4) erstreckenden zentralen Kanal (12) des Hohlwellenmotors gelagert ist und mindestens ein Dichtungsmittel vorgesehen ist, um den zentralen Kanal (12) gasdicht abzudichten,
**dadurch gekennzeichnet,**
**dass** das mindestens eine Dichtungsmittel als in den zentralen Kanal (12) einsetzbares Klappenventil (14) ausgebildet ist, dessen zwei zum distalen Ende des zentralen Kanals (12) weisenden Klappenflügel (14a) unter einem Winkel aneinander anliegen, der flacher ist als der Winkel der Schneide (2a) am distalen Ende des in den zentralen Kanal (12) einsetzbaren Schneidrohres (2).

2. Medizinisches Instrument nach Anspruch 1, **dadurch gekennzeichnet, dass** als zusätzliches Dichtungsmittel am proximalen Ende des zentralen Kanals (9) eine Dichtungskappe (13) festlegbar ist

3. Medizinisches Instrument nach Anspruch 2, **dadurch gekennzeichnet, dass** die Dichtungskappe (13) als vom in den zentralen Kanal (12) einsetzbaren medizinischen Instrument (11) durchstoßbare Membran ausgebildet ist.

4. Medizinisches Instrument nach Anspruch 2, **dadurch gekennzeichnet, dass** in der Dichtungskappe (13) eine durch das in den zentralen Kanal (12) einsetzbaren medizinischen Instrument (11) aufweitbare Öffnung ausgebildet ist.

5. Medizinisches Instrument nach mindestens einem der Ansprüche 2 bis 4, **dadurch gekennzeichnet, dass** die Dichtungskappe (13) als aus einem autoklavierbaren Material bestehendes Einmal-Ventil ausgebildet ist.

6. Medizinisches Instrument nach mindestens einem der Ansprüche 1 bis 5, **dadurch gekennzeichnet, dass** der Außendurchmesser des Schneidrohres (2) im wesentlichen dem Innendurchmesser des zentralen Kanals (12) entspricht.

7. Medizinisches Instrument nach mindestens einem der Ansprüche 1 bis 6, **dadurch gekennzeichnet, dass** das Instrument zur Ausbildung des zentralen Kanals (12) zumindest eine distalseitig angeordnete Trokarhülse (5), einen mit einer zentralen Aufnahme für das anzutreibende Schneidrohr (2) versehenen, als Hohlwellenmotor ausgebildeten Motor (3) sowie eine proximalseitig angeordnete, mit einer zentralen Durchgangsbohrung (1a) versehene Handhabe (1) umfasst.

## Claims

1. Medical instrument for cutting biological tissue, and in particular human tissue, with a hollow cutting tube (2) which can be rotated about its longitudinal axis (4) by a hollow shaft motor (3) and at whose distal end there is at least one cutting edge (2a), and for receiving at least one further medical instrument (8) insertable into the hollow cutting tube (2), which cutting tube (2) is mounted in a central channel (12) of the hollow shaft motor extending along the longitudinal axis (4), and at least one sealing means being provided in order to seal the central channel (12) in a gas-tight manner, **characterized in that** the at least one sealing means is designed as a flap valve (14) which can be inserted into the central channel (12) and whose two flap wings (14a) pointing towards the distal end of the central channel (12) bear on one another at an angle that is shallower than the angle of the cutting edge (2a) at the distal end of the cutting tube (2) insertable into the central channel (12).

2. Medical instrument according to Claim 1,
**characterized in that** a sealing cap (13) can be attached as additional sealing means at the proximal end of the central channel (12).

3. Medical instrument according to Claim 2,
**characterized in that** the sealing cap (13) is designed as a membrane that can be pierced by the medical instrument (11) insertable into the central channel (12).

4. Medical instrument according to Claim 2,
**characterized in that** an opening that can be widened by the medical instrument (11) insertable into the central channel (12) is formed in the sealing cap (13).

5. Medical instrument according to at least one of Claims 2 to 4, **characterized in that** the sealing cap (13) is designed as a valve that can be disposed of after one use and that is made of an autoclavable material.

6. Medical instrument according to at least one of Claims 1 to 5, **characterized in that** the external diameter of the cutting tube (2) corresponds substantially to the internal diameter of the central channel (12).

7. Medical instrument according to at least one of Claims 1 to 6, **characterized in that** the instrument, in order to form the central channel (12), comprises at least one trocar sleeve (5) arranged at the distal end, a motor (3) designed as a hollow shaft motor provided with a central recess for receiving the cutting tube (2) to be driven, and a handle (1) arranged at the proximal end and provided with a central bore (1a).

## Revendications

1. Instrument médical destiné à couper des tissus biologiques et en particulier des tissus humains, comportant un tube de coupe (2) creux, qui peut tourner autour de son axe longitudinal (4) par l'intermédiaire d'un moteur à arbre creux (3) et dont l'extrémité distale porte au moins une lame de coupe (2a), et destiné à recevoir au moins un autre instrument médical (8), qui peut être introduit dans le tube de coupe (2) creux, le tube de coupe (2) étant monté dans un conduit central (12) du moteur à arbre creux, s'étendant le long de l'axe longitudinal (4), et au moins un moyen d'étanchéité étant prévu pour que le conduit central (12) soit rendu étanche aux gaz,
**caractérisé en ce que** ledit au moins un moyen d'étanchéité est réalisé sous la forme d'une vanne papillon (14), qui est apte à être montée dans le conduit central (12) et dont deux ailes (14a), orientées vers l'extrémité distale du conduit central (12), sont agencées en délimitant entre elles un angle, qui est plus aigu que l'angle de la lame de coupe (2a) sur l'extrémité distale du tube de coupe (2) destiné à être monté dans le conduit central (12).

2. Instrument médical selon la revendication 1, **caractérisé en ce qu'**un capuchon d'étanchéité (13), formant un moyen d'étanchéité supplémentaire, peut être fixé contre l'extrémité proximale du conduit central (12).

3. Instrument médical selon la revendication 2, **caractérisé en ce que** le capuchon d'étanchéité (13) est réalisé sous la forme d'une membrane apte à être transpercée par un instrument médical (11) propre à être inséré dans le conduit central (12).

4. Instrument médical selon la revendication 2, **caractérisé en ce qu'**une ouverture extensible est réalisée dans le capuchon d'étanchéité (13) par l'instrument médical (11) propre à être inséré dans le conduit central (12).

5. Instrument médical selon l'une quelconque des revendications 2 à 4, **caractérisé en ce que** le capuchon d'étanchéité (13) est réalisé sous la forme d'une valve à usage unique réalisée dans un matériau pouvant être traité en autoclave.

6. Instrument médical selon l'une quelconque des revendications 1 à 5, **caractérisé en ce que** le diamètre extérieur du tube de coupe (2) correspond sensiblement au diamètre intérieur du conduit central (12).

7. Instrument médical selon l'une quelconque des revendications 1 à 6, **caractérisé en ce que** l'instrument pour la réalisation du conduit central (12) comporte au moins une gaine de trocart (5), agencée du côté distal, un moteur (3) réalisé sous la forme d'un moteur à arbre creux et muni d'un logement central pour le tube de coupe (2) à actionner, ainsi qu'un manche (1) muni d'une forure débouchante (1a) centrale, réalisée du côté proximal.
